# EUROPEAN PATENT SPECIFICATION

(11) **EP 2 047 874 B1**
(45) Date of publication and mention of the grant of the patent: **27.04.2011**
(21) Application number: 09000648.7
(22) Date of filing: 28.09.2000
(51) Int. Cl.: A61M 3/00, A61F 5/445

(54) **Improved colostomy device**
Verbesserte Kolostomievorrichtung
Dispositif de colostomie amélioré

(43) Date of publication of application: 15.04.2009
(62) Divisional of application: 00963123.5
(73) Proprietor: Hollister Incorporated, Libertyville IL 60048 (US)
(72) Inventor: Kim, Jae-Hwang, Suseong-gu Daegu 706-090 (KR)
(74) Representative: Thacker, Darran Ainsley

(56) References cited:
- US-A- 4 662 890
- US-A- 5 569 216
- US-A- 5 860 952

## Description

### Technical Field

The present invention relates,to an improved medical coloctomy device for containing or helping to pass stool. The device is inserted into the rectum of patients who can't control stool output, who are in a coma, or who are bedridden and need long term treatment. The device, if needed, is inserted into the rectum and after a certain package of time ie used, to help pass stool via evacuation through the drainage hose.

### Background art

In general, there are many medical colostomy devices composed of various parts. For example, as the Patent No. 103444 "Medical Colostomy Device Kateta" which was registered by the inventor who invented the present invention shown. A medical colostomy device has the following parts: internal and external balloons disposed on the internal and external sides and the rim of a supporting tube, an extension part which consists of a number of air tubes and round tubes connected with the external balloon.

The extension is disposed on the rim of the external balloon making internal and external balloons. The extension part expands and contrasts freely. An exhausting tube is disposed under the supporting tube forming a thin membrane along the external balloon, and an air tube, and a round tube. A connecting tube is disposed under the internal and external balloons and a supply tube which goes through the connecting tube.

Accordingly, the above medical colostomy device takes an important role in managing patients who can't control stool output, or who are in a coma or who need long term treatment as the result of a stroke and cannot control bowel function or who need an enema. But there are some problems with the device when placed in the rectum such as the internal and external balloons being pushed into the upper part of rectum, the leakage of liquids and gases as the supplying tube for injection and air and connecting tube are connected separately with the outside of exhausting tube so the gap between rectum and anus can not be closed fully, and the accumulation of liquids and gases from the rectum in the space between the anus and drainage hose.

Another example, as the Utility Model No. 109896 "Medical Colostomy Device" which was registered by the inventor who invented the present invention shown, a medical colostomy device forms the supplying tube and the internal tube as an integration disposed on the internal and external tubes, a connecting tube with a joint tube under the external tube, a holder on the angled part of the supplying tube, an air supplying tube, an injection oupplying tube, a washing fluid supplying tube connected with the internal and external tubes of above supplying tube, connecting socket can be inserted and installed connecting with washing fluid case, air cylinder and supplying tube.

Accordingly, the above device is effective for patients, who have colostomy on their abdomens after rectum cancer surgery etc. or who should be on long-term bed rest, without any inconvenience or bad effects. It is, however, uncomfortable for patients whose anuses are worn-out from having loose bowels, who are incontinent or who are stricken with paralysis.

Another example, as the Utility Model No. 118334 "Tubes of Medical Colostomy Device" which was registered by the inventor who invented the prevent invention shown, in composing the internal wall thickly with elastic material to maintain the original shape of the vacant slab, when extending under the lower side of the external balloon in which the vacant slab is formed, making it air-tight is successful when expanding internal and external balloons.

For the above case, even though air is supplied for expending the external balloon, making it air-tight is achieved perfectly without any gap, as it contacts with the external tube closely when expansion of the internal balloon is formed inside the external balloon.

But the tubes are pushed into the upper rectum and the leakage of liquids and gases happens as tubes for air supplying are located outside of that exhausting tube.

The above device can hurt a patient's body as the edges of the supporting tube formed between the internal and external tubes rub against the inner walls and it's difficult to insert into the patients with small anuses.

US 5 569 216 (Kim) discloses a colostomy device that is suitable for stomal and rectal use. A first device include external and internal balloons 100, 110. A supporting tube 118 is disposed between the external and internal balloons. A drainage base 102 is provided. An air supply tube 108 is used to supply air to the external and internal balloons. An annular supporting plate 104 is used to fix the device relative to the abdominal wall of the stoma and acts as a fixing aid. A second device is for rectal use and does not include the annular supporting plate.

US 4662890 (Burton) discloses a colostomy device for stomal use. The device includes an external balloon 14. An air supply tube 16 is used to supply air to the external balloon 14. A plate 44 includes a pair of flexible straps that are used to retain an external tube 42 in a doubled-back configuration.

### Disclosure of Invention

Accordingly, it is the object of the present invention as claimed in claim 1 to eliminate the above problems, to help control bowel output and enemas easily, and to prevent the tubes from being pushed into the upper part of the rectum making it perfectly air-tight when in use. This device can be used by patients who cannot control bowel movements, those who are bedridden, those who have frequently loose bowels with worn-out anuses, and those who are stricken with paralysis.

Another object of the present invention is to insert a colostomy device into the anus without causing any damage on the inner wall, especially for patients who have small anuses.

To achieve the above object when composing a colostomy device with internal and external balloons and drainage hose, the fixing aid is disposed on the external side of the drainage hose which is a little distant from the tubes, and the supply tube for supplying air to the internal and external balloons is connected to the internal and external balloons, through the inside of the drainage hose.

For the above fixing aid, a holder made from elastic material is adhered and composed on the external side of the drainage hose and a detachable duplex adhesive plate is disposed on the whole side of the holder.

In executing the present invention, air ring can be used for a holder and a fixing band disposed long on both sides is used with a holder of which the width is composed narrowly.

A colostomy device with the above composition can be selected and used according to the conditions of applicable patients.

Also in the present invention, a protection cover made from soft and elastic material such as silicon and disposed on the rim of the external side of the support tube composing a colostomy device covers angles of the support tube.

After letting out air from the internal and external balloon, the colostomy device is folded by half and is easily inserted into the anus with a protective cover wrapped at the front end part.

### Brief Description of the Drawings

FIG 1 is a side view of the present invention
FIG 2 is a side view of the other exemplified figure
FIG 3 is a side, view of the other exemplified figure
FIG 4 is a conditional view of the duplex adhesive plate of the present invention
FIG 5 is a conditional view of the duplex adhesive plate of the present invention
FIG 6 is a side view of the other exemplified figure
FIG 7 is a side view of FIG 6
FIG 8 is a partial cross-sectional view of the other exemplified figure
FIG 9 is an exemplifying view of the present invention for inserting the colostomy device easily.

Descriptions of major parts of this invention as shown on the drawings.
(2) Colostomy device
(4) External balloon
(6) Internal balloon
(8) Drainage hose
(10) Air supply tube
(11) Fixing band
(12) Holder
(14) Duplex detachable plate
(15) Support tube
(16) Air ring
(17) Protection cover
(18) Check valve
(19) Protection membrane

### Best Mode for Carrying out the Invention

Detailed descriptions of the present invention are provided as follows according to the attached drawings.

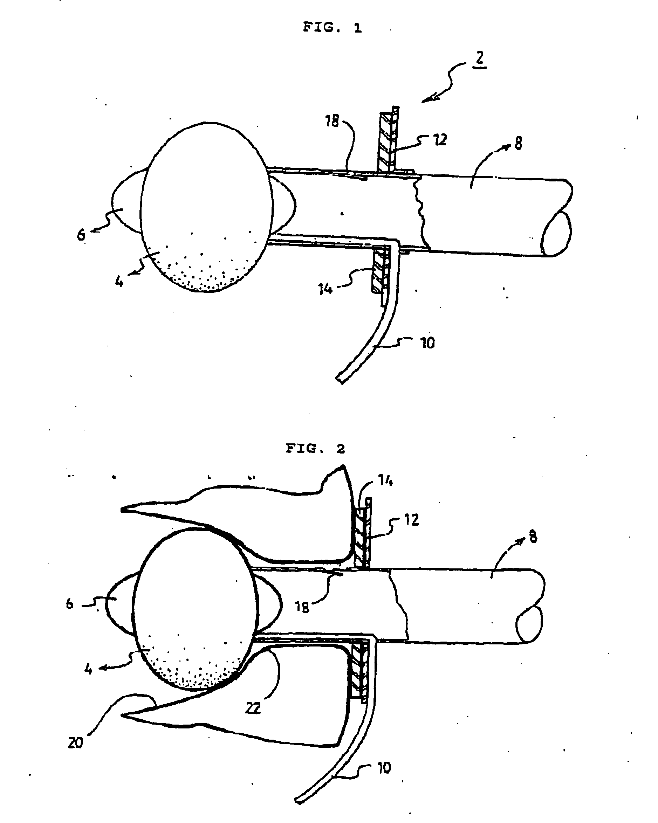

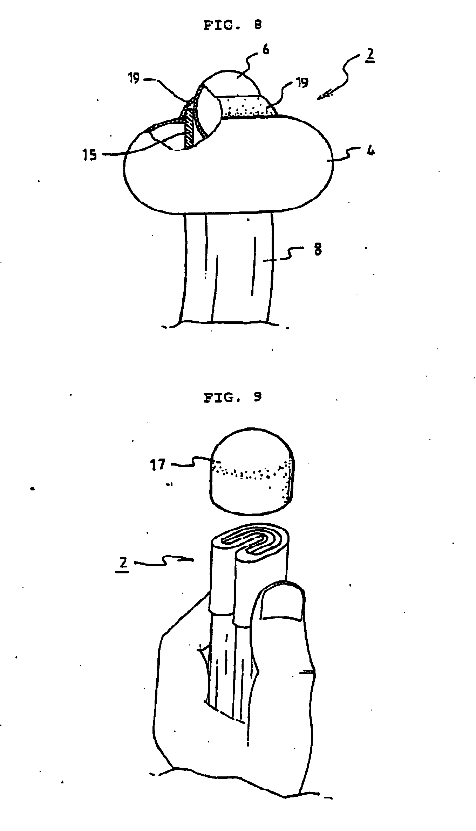

FIG 1 shows a colostomy device (2) having a ring configured external balloon (4), an elastic thin membrane, an internal balloon (6) disposed within the external balloon (4), a supporting tube (15) (FIG 8) between the external and internal balloon and a drainage hose (8). A fixing aid or holder (12) is provided on the external side of the drainage hose (8) (i.e. that part of the drainage hose (8) that lies outside the rectum in use) for preventing the internal and external balloons (4) and (6) from being pushed into the upper part of the rectum when the colostomy device (2) is used.

In FIG 1 the holder (12) is adhered on the external side of the drainage hose (8) as an integration type by a thin silicon-like material and connects a round detachable duplex adhesive plate (14) to the whole side.

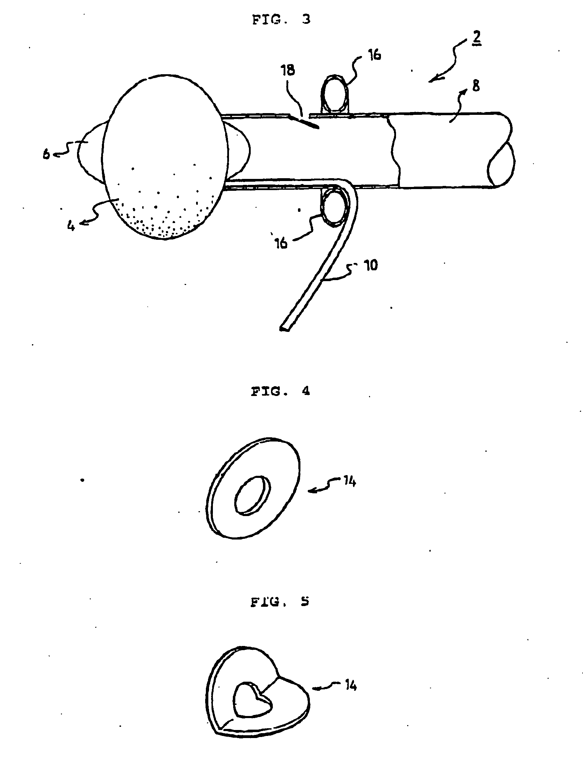

An air ring (16) can be used as an alternative to the holder (12) as shown in FIG. 3.

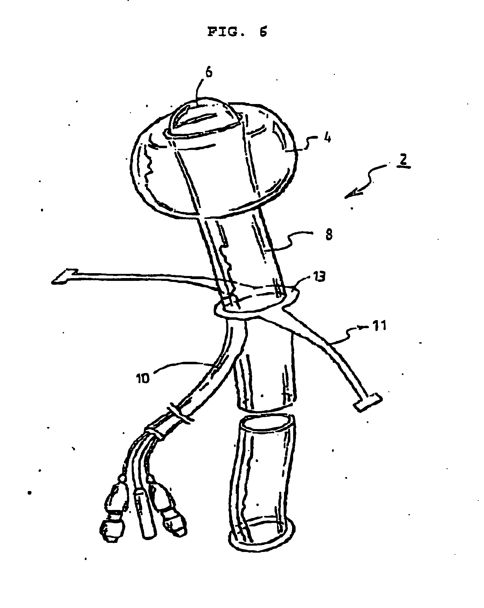

Furthermore, a narrow holder (13) can be adhered narrowly as an integration type on the external side of the drainage hose (8) as shown in FIG 6. The holder (13) includes a fixing band (11) disposed long on both end sides that can be used to attach the colostomy device (2) to the patient's anus.

To prevent leakage of liquids and gases through the inserted part while using the device in the present invention, the air supply tube (10) which is for supplying or exhausting air to/from the internal and external balloons (4) and (6) is connected to the internal and external balloons (4) and (6) through the inside of the drainage hose (8) after passing through the thin holder (13).

A check valve (18) which runs one way only from the outer drainage hose (8) to the inner drainage hose (8) is provided on the drainage hose (8) between the external balloon (4) and the fixing aid.

The edges of the supporting tube (15) can be covered with a protection membrane (19). This is formed from a silicon-like soft and elastic material which is composed on the rim of the supporting tube (15) between the internal and external balloons (4) and (6) as shown in FIG 8. The colostomy device (2) can then be easily inserted without any damage to the inner walls of the anus.

It is difficult to insert the internal balloon (6) with air supplied (i.e, inflated), so the internal and external balloons (4) and (6) can be deflated as shown in FIG 9. The colostomy device (2) is folded in half lengthwise for volume reduction so that it can easily be inserted into the anus with a protection cover (17) made from soft material wrapped around the front end part. After completing installation, the internal and external balloons (4) and (6) are supplied with air and become inflated. The protection cover (17) can be taken off and is exhausted out during bowel movement or injection.

The present invention composed as above, is for containing bowel movements or helping to pass stools, installing it in the rectum of the patients who can't control bowel movements, who are in a coma or who are bedridden and need long term treatment. The device if needed, is used conveniently for enemas and works in the following manner.

FIG 2 shows the colostomy device (2) installed and used on the anus of a patent. The fixing aid is adhered on the external side of the drainage hose (8) and contacts the anus of the patient supporting it to prevent the internal and external balloons (4) and (6) from being pushed into the upper part of rectum (20) when the colostomy device (2) is used.

When a holder (12) is used as the above fixing aid, it provides an air-tight seal since the duplex adhesive plate (14) is inserted on the whole side of the holder (12) and adhered around the anus.

The duplex adhesive plate (14) with free bends features excellent air-tight properties since the shape changes and adapts to the shape of the anus.

The material for the duplex adhesive plate (14) should be elastic and harmless to people without causing any adverse skin reaction. When an air ring (16) is used as the fixing aid as shown in FIG 3, the air ring (16) prevents leakage of liquids and gases from the rectum, adhering the external tube closely to the wall of the rectum during injection.

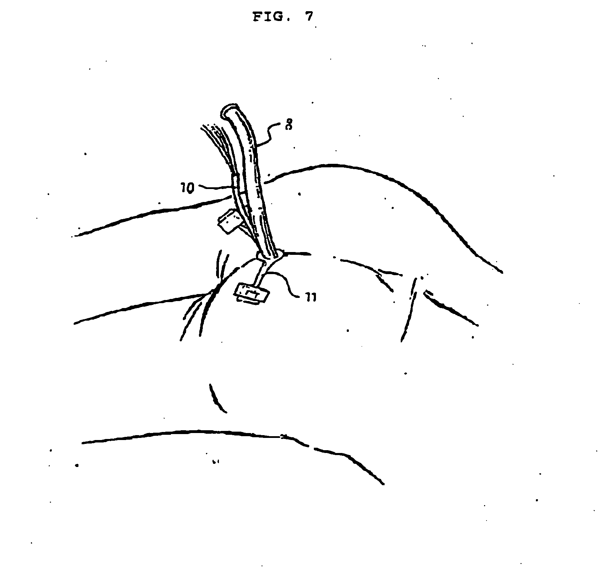

The adhesive plate (14) is impossible to use for patients who have inflammation and skin damage, so in this situation the fixing band (11) with adhesive part composed on the end part of the band as shown in FIG 6 should be used. When using the fixing band (11), the position of the colostomy device (2) can be fixed adhering around the anus of the patient as shown in FIG 7.

When the colostomy device (2) provided in the present invention, is installed in the rectum (20) located at the anus of a patient as shown in FIG 2, there is no leakage of liquids and gases and no gap between the drainage hose (8) and the anus since the air supply tube (10) is inserted inside the drainage hose (8).

The drainage hose (8) located inside the anal tube with check valve (18) which runs one way only from the outer drainage hose (8) to the inner drainage hose (8) prevents the leakage of liquids and gases.

The part of the drainage hose (8) formed on the external side of the fixing aid (i.e. extending outwards beyond the fixing aid in use) can be removed if necessary, especially in the case of ambulatory patients, using it during injection and bowel movement without any inconvenience.

### Industrial Applicability

Accordingly, the present invention relates to a colostomy device which can be used for incontinent patients and/or for movement with the following positive effects; preventing the internal and external balloons from being pushed into the upper part of the rectum by the fixing aid while using the device, preventing the leakage of liquids and gases, protecting the epidermis around the anus by the check valve of the drainage hose which drains any liquids or gases that enter the space between the anal tube and drainage hose, and facilitating easy installation of the colostomy device.

## Claims

1. A colostomy device (2) insertable into the anus without causing damage to the inner wall of the anus, the device comprising external and internal balloons (4,6), the internal balloon (6) being disposed within the external balloon (4), a supporting tube (15) between the external and internal balloons (4, 6), a drainage hose (8), an air supply tube (10) connected to the external and internal balloon (4, 6) and a fixing aid on an external side of the drainage hose for preventing the device from being pushed into an upper part of the rectum (20) when the device is used, and **characterised in that** the supporting tube (15) is foldable when the external balloon (4) is deflated so that it can be easily inserted into the anus of a patient.

2. The device (2) of claim 1, wherein the supporting tube is foldable by half lengthwise when the external balloon (4) is deflated.

3. The device (2) of claim 1 or claim 2, further comprising a protection cover (17) made from a soft material wrapped around a front end part of the colostomy device (2).

4. The device (2) of claim 3, wherein the protection cover (17) is removable from the front end part of the colostomy device (2) upon expansion of the external balloon (4).

## Patentansprüche

1. Eine Kolostomievorrichtung (2), die in den Anus eingeführt werden kann, ohne eine Beschädigung der inneren Wand des Anus hervorzurufen, wobei die Vorrichtung umfasst: externe und interne Ballons (4, 6), wobei der interne Ballon (6) innerhalb des externen Ballons (4) angeordnet ist, eine Stützröhre (15) zwischen den externen und internen Ballons (4, 6), einen Drainageschlauch (8), eine Luftzufuhrröhre (10), die mit dem externen und dem internen Ballon (4, 6) verbunden ist, und eine Befestigungshilfe auf einer externen Seite des Drainageschlauchs, um die Vorrichtung daran zu hindern, in einen oberen Teil des Rektums (20) gedrückt zu werden, wenn die Vorrichtung verwendet wird, **dadurch gekennzeichnet, dass** die Stützröhre (15) faltbar ist, wenn der externe Ballon (4) nicht aufgeblasen ist, so dass sie einfach in den Anus eines Patienten eingeführt werden kann.

2. Vorrichtung (2) nach Anspruch 1, bei der die Stützröhre in der Längsrichtung um die Hälfte faltbar ist, wenn der externe Ballon (4) nicht aufgeblasen ist.

3. Vorrichtung (2) nach Anspruch 1 oder 2, weiterhin eine Schutzabdeckung (17) aus einem weichen Material umfassend, welches um ein vorderes Endteil der Kolostomievorrichtung (2) gewickelt ist.

4. Vorrichtung (2) nach Anspruch 3, bei welcher die Schutzabdeckung (17) von dem vorderen Endteil der Kolostomievorrichtung (2) nach dem Aufblasen des externen Ballons (4) entfernbar ist.

## Revendications

1. Dispositif de colostomie (2) insérable dans l'anus sans causer de dommage à la paroi interne de l'anus, le dispositif comprenant des ballonnets externe et interne (4, 6), le ballonnet interne (6) étant disposé à l'intérieur du ballonnet externe (4), un tube de support (15) entre les ballonnets externe et interne (4, 6), un tuyau de drainage (8), un tube d'alimentation en air (10) raccordé aux ballonnets externe et interne (4, 6) et un dispositif auxiliaire de fixation sur un côté externe du tuyau de drainage pour empêcher le dispositif d'être poussé dans une partie supérieure du rectum (20) lors de l'utilisation du dispositif, et **caractérisé en ce que** le tube de support (15) est repliable lorsque le ballonnet externe (4) est dégonflé de sorte à pouvoir être inséré facilement dans l'anus d'un patient.

2. Dispositif (2) selon la revendication 1, dans lequel le tube de support est repliable de moitié dans le sens de la longueur lorsque le ballonnet externe (4) est dégonflé.

3. Dispositif (2) selon la revendication 1 ou la revendication 2, comprenant en outre un cache de protection (17) en un matériau souple entourant une partie d'extrémité avant du dispositif de colostomie (2).

4. Dispositif (2) selon la revendication 3, dans lequel le cache de protection (17) peut être retiré de la partie d'extrémité avant du dispositif de colostomie (2) lors de la dilatation du ballonnet externe (4).
